# EUROPÄISCHE PATENTSCHRIFT

(11) **EP 3 363 402 B1**
(45) Veröffentlichungstag und Bekanntmachung des Hinweises auf die Patenterteilung: **20.05.2020**
(21) Anmeldenummer: 18157849.3
(22) Anmeldetag: 21.02.2018
(51) Int. Cl.: A61B 90/98, A61M 1/00, A61B 1/00, G06K 19/073

(54) **MEDIZINVORRICHTUNG UND MEDIZINISCHES SYSTEM**
MEDICAL DEVICE AND MEDICAL SYSTEM
DISPOSITIF MÉDICAL ET SYSTÈME MÉDICAL

(30) Priorität: 21.02.2017 DE 102017103511
(43) Veröffentlichungstag der Anmeldung: 22.08.2018
(73) Patentinhaber: Karl Storz SE & Co. KG, 78532 Tuttlingen (DE)
(72) Erfinder: Bechtel, Christin, 78532 Tuttlingen (DE); Schwarz, Peter, 78532 Tuttlingen (DE)

(56) Entgegenhaltungen:
- EP-A1- 3 315 080
- WO-A1-2017/023735
- US-A1- 2004 215 131
- US-A1- 2010 079 289
- US-A1- 2012 280 044
- US-A1- 2016 235 916

## Beschreibung

Die Erfindung betrifft eine Medizinvorrichtung, welche insbesondere ein Bestandteil eines medizinischen Systems ist, mit einer Schalteinrichtung zum Aktivieren oder Deaktivieren einer elektrischen Einrichtung des medizinischen Systems, wobei die Schalteinrichtung ein erstes Schaltelement und ein zweites Schaltelement aufweist, welche zueinander beweglich ausgestaltet sind, sodass eine erste Schalterstellung und eine zweite Schalterstellung realisierbar sind und ein medizinisches System mit einer elektrischen Einrichtung und der Medizinvorrichtung.

Medizinisches Fachpersonal muss häufig beim Bedienen medizinischer Handhabungsgeräte, wie beispielsweise Endoskope oder Sauger, unterstützende oder zusätzliche medizinische Geräte bei der Behandlung eines Patienten definiert schalten.

Hierzu schlägt die DE 10 2014 105 509 A ein Bediengerät zum Steuern einer medizinischen Einrichtung vor, mit einem Gerätekörper und mindestens einem druckbetätigten Schaltelement, mit dem sich die medizinische Einrichtung steuern lässt.

In der WO 2016/008041 A1 ist eine um den Finger angeordnete Fernsteuerung offenbart, mit welcher medizinische Einrichtungen gesteuert werden können.

Auch Vorrichtungen zum Fernsteuern mehrerer elektronischer Geräte sind bekannt. So offenbart die US 8,638,191 B2 ein System zum kabellosen Fernsteuern einer oder mehrerer Vorrichtungen.

Kabellose Bedienelemente zum Steuern eines chirurgischen Gerätes oder eines optischen Beobachtungs-gerätes werden in der DE 102 45 501 A1 offenbart.

Sogenannte RFID-Technologien (RFID ist eine englischsprachige Abkürzung und steht für radio-frequency identification) haben Einzug in die Medizintechnik gehalten, da damit ein kabelloser Informationsaustausch möglich ist. Beispielsweise sei an dieser Stelle die DE 10 2013 101 158 A1 genannt.

RFID-Technologien sind beispielsweise in der DE 10 2011 122 797 A1 offenbart, wobei darin eine Karte aus Kunststoff und/oder Papier mit einem integriertem RFID-Transponder dargestellt ist, der einen Chip mit einer Speichereinheit und eine Spule aufweist, welche als Flachspule auf Substrat angeordnet ist.

In der US 7,762,471 B2 ist eine Transponderkarte offenbart, welche Informationen über verschiedene Accountnummern einer Bank oder Kreditkarte enthält.

Auch sind funkgesteuerte Karten bekannt deren äußere Hülle ein versiegeltes Gehäuse für interne elektronische Komponenten bildet, und in die ein RFID-IC, welcher einen Datenspeicher und einen Radiofrequenztransmitter enthält, offenbart ist(US 6,863,220 B2).

In der US 2007/0290051 wird eine Antenne für EC-/Kreditkarten beschrieben, welche mindestens ein integriertes RFID-Chipmodul und eine damit verbundene Antenne enthalten. Auch ist eine in einem Gehäuse integrierte drahtlose Identifikationsmöglichkeit bekannt, welche auf ein Anfragesignal antwortet. Siehe hierzu US 6,693,513 B2.

Weiterhin ist in der DE 10 2007 053 733 A1 eine Vorrichtung zur Aufnahme von Sensorsignalen beschrieben, welche einen Reader aufweist, der Energie in Form eines elektromagnetischen Frequenzfeldes senden und/oder empfangen kann, und einen Transponder, der die Energie des Readers empfangen und zum Teil in elektrische Energie umwandeln kann, wobei der Transponder mindestens mit einem Sensor verbunden ist.

In der US 7,098,794 B2 ist ein deaktivierbares Sicherungssystem offenbart. Verschiebbare Schutzhüllen sind beispielsweise in der US 7,523,870 B2 beschrieben, welche eine Sicherheitskarte mit RFID-Element schaltbar freigeben.

Um RFID-Elemente beispielsweise auf Sicherheitskarten und dergleichen freizugeben, sind An- und Ausschaltvorrichtungen beispielsweise in der US 8,844,831 B2 und US 9,17,154 B2 beschrieben, wobei jeweils das RFID-Element selbst "freigeschaltet oder deaktiviert" wird. Auch sind Ausweishüllen mit integrierten Sicherheitsausweisen, welche RFID-Elemente aufweisen, bekannt (US 9,135,548 B2).

In der US 2016/0235916A1 ist eine Vorrichtung zur Medikamentengabe offenbart, mit einem Schalter, der bei Betätigung zusätzlich ein RFID-Element abschirmt und damit ein Signal blockiert, was einer weiteren Vorrichtung mitteilt, dass ein Schaltvorgang erfolgt ist. Das Blockieren des RFID-Signals aktiviert jedoch nicht die Vorrichtung zur Medikamentengabe, sondern dies erfolgt über den konventionellen Schalter, der direkt an der Vorrichtung befestigt ist. Das RFID-Signal dient nur der Kontrolle oder Überwachung.

Mit dem vorliegend beschriebenen Stand der Technik ist es nicht möglich medizinische Geräte dezidiert anzusteuern und zu schalten, ohne zusätzliche beispielsweise an der Hand oder an den Fingern zu tragende Geräte oder zusätzliche Schalter zu verwenden. Insbesondere sind diese Schalter nicht autoklavierbar. Das heißt ein Sterilisieren der Fernsteuerung und dergleichen ist nicht möglich oder aufgrund von Demontiertätigkeiten sehr aufwändig. Zumeist muss ein Auseinanderbauen erfolgen, oder es müssen ganze Einheiten als solche nach der erstmaligen Verwendung entsorgt werden.

Aufgabe der Erfindung ist es den Stand der Technik zu verbessern.

Gelöst wird die Aufgabe durch eine Medizinvorrichtung, gemäß Anspruch 1, welche insbesondere ein Bestandteil eines medizinischen Systems ist, mit einer Schalteinrichtung zum Aktivieren oder Deaktivieren einer von der Medizineinrichtung räumlich beabstandeten elektrischen Einrichtung des medizinischen Systems, wobei die Schalteinrichtung ein erstes Schaltelement und ein zweites Schaltelement aufweist, welche zueinander beweglich ausgestaltet sind, sodass eine erste Schalterstellung und eine zweite Schalterstellung realisierbar sind, und das erste Schaltelement einen Teil eines faradayschen Käfigs oder einen faradayschen Käfig und das zweite Schaltelement eine Sendeeinrichtung aufweist, wobei das erste Schaltelement und das zweite Schaltelement derart zueinander ausgebildet sind, dass in der ersten Schalterstellung ein Aktivieren oder Deaktivieren der elektrischen Einrichtung des medizinischen Systems und in der zweiten Stellung ein zu der ersten Schalterstellung komplementäres Deaktivieren oder Aktivieren durch ein Abschirmen der Sendeeinrichtung mittels des Teils des faradayschen Käfigs oder mittels des faradayschen Käfigs erfolgt.

So kann beispielsweise eine Medizinvorrichtung bereitgestellt werden, welche einen Schalter aufweist, mit dem eine elektrische Einrichtung eines medizinischen Systems beschaltbar ist. Die elektrische Einrichtung ist dabei räumlich von der Medizinvorrichtung und dem Schalter getrennt und aus der Entfernung beschaltbar, wobei die Schalteinrichtung in Art einer Fernbedienung wirkt.

Beispielsweise kann es sich bei der Medizinvorrichtung um einen Haltegriff für einen medizinischen Sauger oder den Sauger selbst handeln, welcher einen Schiebegriff aufweist, welcher durch einen Finger bedienbar ist. Durch das Verschieben wird dann die Sendeeinrichtung freigegeben, wobei während der ganzen Zeit ein mögliches Signal der Sendeeinrichtung abgefragt wird. Sobald das Sendesignal der Sendeeinrichtung durch das medizinische System abfragbar oder empfangbar ist, wird entsprechend die elektrische Einrichtung des medizinischen Systems an- oder abgeschaltet.

Wird durch den faradayschen Käfig das Senden blockiert, empfängt das medizinische System kein Signal, und es wird die elektronische Einrichtung invers geschaltet.

Insbesondere wenn es sich um eine passive Sendeeinrichtung handelt, kann diese frei von einer Energieversorgung sein. In diesem Fall weist die Vorrichtung keine Energiequelle und/oder Kabel auf, sodass die Medizinvorrichtung mit Schalteinrichtung autoklavierbar ist.

Somit kann ein medizinisches Instrument, welches keine elektrischen Komponenten aufweist, wie dies häufig der Fall ist, einen elektromagnetisch wirkenden Schalter aufweisen, wobei das medizinische Instrument trotzdem zusammen mit dem Schalter autoklavierbar oder gut reinigbar ist.

Folgendes Begriffliche sei erläutert.

Eine "Medizinvorrichtung" ist insbesondere ein medizinisches Handhabungsgerät oder ein medizinisches Instrument, welches manuell durch einen Bediener betätigt wird. Dabei kann es sich um einen Sauger, Endoskop, oder auch um ein Skalpell und dergleichen handeln. Insbesondere ist ein Griffstück von dem Begriff umfasst, an welches beispielsweise ein medizinisches Gerät, wie beispielsweise eine Pumpe, und/oder ein medizinisches Instrument wie beispielsweise der Sauger angeflanscht werden kann. Dieses Griffstück ist insbesondere ergonomisch auf den Bediener angepasst und kann somit unterschiedlichste Werkzeuge und Hilfsmittel bedienen.

Ein "medizinisches System" umfasst zum einen die Medizinvorrichtung, und zum anderen ein medizinisches Gerät. Das medizinische Gerät kann dabei beispielsweise eine Saugpumpe sein oder ist beispielsweise eine Kamerakontrolleinheit für Endoskope, eine Ergänzung zu einem Zahnarztstuhl oder ein bildgebendes Gerät, wie eine Röntgenapparatur oder eine Ultraschalleinheit.

Eine "Schalteinrichtung" ist insbesondere ein Schalter, welcher wenigstens zwei Zustände annehmen kann. Bei zwei Schaltzuständen kann es sich beispielsweise um die Zustände "Ein" und "Aus" handeln. In diesen beiden Schaltzuständen wird in dem einen Fall die "Sendeeinrichtung" "frei" gegeben und in dem anderen Fall blockiert. Im freigegebenen Fall sendet die Sendeeinrichtung ein Signal an das medizinische Gerät, welches dieses Signal entsprechend der internen Logik in ein Schaltsignal für die "elektrische Einrichtung" aufbereitet und dieser zuführt. Beim Blockieren kann ein Sendesignal der Sendeeinrichtung nicht an das medizinische Gerät gesandt werden, sodass das medizinische Gerät, entsprechend der inneren Logik die elektrische Einrichtung entsprechend invers beschaltet. Der Schalter kann beispielsweise als Kippschalter ausgestaltet sein.

Die "elektrische Einrichtung" kann im einfachsten Fall ein elektronisches Speicherelement sein, welches beispielsweise einen Zustand wie 0 oder 1 abspeichert, welcher durch einen Rechner und dergleichen abfragbar und in ein Steuersignal oder Regelsignal überführbar ist. In sehr einfachen Fällen kann die elektronische Einrichtung auch ein mechanisches Bauteil oder ein motorisches Bauteil sein. Beispielsweise kann eine Klappe oder ein Abfluss elektronisch auf- oder zugeschaltet werden, oder eine elektrische Maschine, wie beispielsweise eine Saugpumpe, an- oder ausgeschaltet werden. Auch kann zwischen zwei unterschiedlichen Geräten oder Geräteeinheiten geschaltet werden. So kann bei einer Saug-Spülpumpe zwischen der Saug- und Spülfunktion geschaltet werden. Aus dem Stand der Technik ist bekannt, eine Saugpumpe permanent aktiviert zu lassen und das Saugen durch Abdecken und Freigeben eines Unterbrecherlochs am Sauginstrument mit dem Finger zu unterbrechen oder fortzusetzen. Dies hat jedoch den Nachteil, dass andauernd ein Geräusch erzeugt wird, auch wenn die Saugung gar nicht benötigt wird. Wird mit dem vorstehend beschriebenen Schalter beispielsweise eine Pumpe "ausgeschaltet", so wird vorteilhafter Weise auch die Geräuschbelastung reduziert

Unter "Aktivieren" oder "Deaktivieren" ist jeweils das alternative Schalten in einen der Zustände zu verstehen.

Das "erste Schaltelement" und das "zweite Schaltelement" realisieren in Wechselwirkung zueinander die eigentliche Schalteinrichtung. So kann eines der beiden Schaltelemente fest und im Bezug zu diesem festen Schaltelement das jeweils andere Schaltelement beweglich ausgestaltet sein. Auch können beide Schaltelemente beweglich ausgestaltet sein, sodass diese beispielsweise zueinander verdreht werden. Wesentlich ist, dass durch die Bewegung des Schaltelements oder der Schaltelemente in einem ersten Zustand die Sendeeinrichtung freigegeben wird, sodass ein Signal der Sendeeinrichtung zum medizinischen Gerät ausgesandt werden kann, und in dem anderen Schaltzustand das Senden zum medizinischen Gerät unterbrochen wird. So weist beispielsweise der erste Schalter die Sendeeinrichtung auf und der zweite Schalter ist wenigstens teilweise als faradayscher Käfig ausgebildet. Durch das Bewegen der beiden Schaltelemente wird beispielsweise der faradaysche Käfig des zweiten Schalters um die Sendeeinrichtung herum angeordnet, sodass etwaig ausgesandte Signale der Sendeeinrichtung nicht außerhalb des faradayschen Käfigs gelangen können. Auch kann das erste Schaltelement einen Teil des faradayschen Käfigs aufweisen, sodass beispielsweise unter Verwendung des zweiten Schaltelements ein vollständiger faradayscher Käfig realisiert wird.

Ein "faradayscher Käfig" (auch Faraday-Käfig genannt) ist eine geschlossene Hülle aus elektrischen Leitern. Bei diesen Leitern kann es sich um ein Drahtgeflecht oder auch ein Blech handeln. Der elektrische Leiter bewirkt dabei eine elektrische Abschirmung, welche vorliegend verhindert, dass ein Sendesignal der Sendeeinrichtung zum medizinischen Gerät gelangen kann.

"Beabstandet", "aus der Ferne" und andere hier verwendete Formulierungen bedeuten aus einigen Zentimetern oder einigen Metern Entfernung, je nach Ausgestaltung des verwendeten RFID-Elements und wie dies im Rahmen der an sich bekannten RFID-Technologie möglich ist.

In einer weiteren Ausführungsform ist die Sendeinrichtung frei von einer Energieversorgung.

Somit werden Batterien oder Akkus für die Sendeeinrichtung nicht benötigt. Auch eine externe Energiezufuhr mittels Kabel kann somit entfallen. Dies kann beispielsweise mittels eines passiven RFID-Elements als Sendeeinrichtung realisiert werden. RFID ist eine englische Abkürzung und steht für radio-frequency identification. Dies bedeutet eine Identifizierung mithilfe elektromagnetischer Wellen.

Insgesamt wird darunter eine Technologie für Sender- Empfängersysteme zum automatischen und berührungslosen Identifizieren und Lokalisieren von Objekten und Lebewesen mittels Radiowellen verstanden. Ein RFID-System besteht aus einem Transponder (umgangssprachlich auch Funketikett genannt) welcher sich beispielsweise an einem Gegenstand oder Lebewesen befindet und einen kennzeichnenden Code enthält. Diese Einheit wird vorliegend als RFID-Element bezeichnet. Diesem zugeordnet ist meist ein Lesegerät, welches die Kennung des RFID-Elements ausliest. Das Lesegerät ist vorliegend insbesondere dem medizinischen Gerät zugeordnet und übermittelt dementsprechend detektierte Informationen an das medizinische Gerät, oder kann diese bereits schon ausgewertet und verarbeitet an die elektrische Einrichtung als Steuersignal weiterleiten. Die Kommunikation des Lesegerätes mit dem medizinische Gerät kann kabelbasiert oder auch funkbasiert erfolgen, sodass das Lesegerät frei im Raum anordenbar ist.

Grundsätzlich weist ein RFID-Element eine Antenne, einen analogen Schaltkreis zum Empfangen und Senden, und einen digitalen Schaltkreis, sowie einen permanenten Speicher auf. Teilweise ist der Speicher nur einmal beschreibbar, kann aber jedoch auch teilweise überschrieben werden.

Die grundsätzliche Funktionsweise ist wie folgt: Das Lesegerät, das je nach Typ gegebenenfalls auch Daten schreibt, erzeugt ein hochfrequentes elektromagnetisches Wechselfeld, dem das RFID-Element ausgesetzt wird. Die von ihm über die Antenne aufgenommene Hochfrequenzenergie, dient während des Kommunikationsvorgangs beispielsweise als Stromversorgung für einen Chip des RFID-Elements. Bei aktiven RFID-Elementen kann die Energieversorgung auch durch eine eingebaute Batterie erfolgen. Der so aktivierte Mikrochip im RFID-Element decodiert die vom Lesegerät gesendeten Befehle. Die Antwort codiert und moduliert das RFID-Element in das eingestrahlte elektromagnetische Feld durch Feldschwächung im kontaktfreien Kurzschluss oder gegenphasige Reaktion des vom Lesegerät ausgesendeten Feldes. Damit überträgt das RFID-Element seine eigene (unveränderliche) Seriennummer, weitere Daten des gekennzeichneten Objekts oder andere vom Lesegerät abgefragte Informationen. In diesem Fall erzeugt das RFID-Element selbst kein eigenes Feld, sondern beeinflusst das elektromagnetische Sendefeld des Readers. Vorliegend wird diese Funktion jedoch auch als "Senden" verstanden, da durch Wechselwirkung des RFID-Elements eine Information an das Lesegerät übermittelt wird.

Allgemein gilt, dass passive RFID-Elemente sich aus den Funksignalen des Lesegerätes mit Energie versorgen. Mit einer Spule als Empfangsantenne wird beispielsweise durch Induktion ähnlich wie in einem Transformator ein Kondensator aufgeladen, der es ermöglicht, die Antwort während Unterbrechungen des Abfragesignals zu senden. Dies ermöglicht einen empfindlichen Empfang des Antwortsignals ungestört von Reflektionen des Abfragesignals von anderen Objekten. Vorliegend können auch semiaktive RFID-Elemente und/oder auch semipassive RFID-Elemente verwendet werden, welche insbesondere ihre Rückstreukoeffizienten modellieren. RFID-Elemente können sowohl Langwellen, Kurzwellen als auch UHF- oder Mikrowellenfrequenzen verwenden.

Aufgrund der Eigenschaft, dass eine Codierung des RFID-Elements übertragbar ist, kann vorliegend die Medizinvorrichtung eindeutig identifizierbar sein. Insbesondere wenn mehrere Handhabungsgeräte eingesetzt werden, kann über das Codieren ein medizinisches Gerät mehrere unterschiedliche Handhabungsgeräte schalten. Beispielsweise durch ein inkrementelles Zählen und Überschreiben des jeweiligen Speichers im RFID-Element kann die maximale Einsatzfähigkeit begrenzt werden. Insbesondere in medizinischen Anwendungen kann dies sehr wichtig sein. Auch kann gewährleistet werden, dass ein derartiger Schalter oder ein derartiges medizinisches Handhabungsgerät nur einmal eingesetzt wird.

Besonders bevorzugt ist, dass die Sendeeinrichtung autoklavierbar ausgestaltet ist, sodass nach einem Autoklavieren der Medizinvorrichtung die Sendeeinrichtung weiterhin funktionsgemäß und steril einsetzbar ist.

Insbesondere durch Verwenden von passiven RFID-Elementen kann dies realisiert werden. Das Autoklavieren erfolgt insbesondere mittels eines Autoklaven bei dem die zu autoklavierenden Gegenstände in einen verschließbaren Druckbehälter eingebracht und thermisch im Überdruckbereich behandelt werden. Vorliegend kann somit ein Sterilisieren der medizinischen Vorrichtungen und somit der medizinischen Handhabungseinrichtungen realisiert werden. Die üblichen Temperaturen beim Sterilisieren und somit vorliegend auch beim Autoklavieren liegen zwischen 60°C und 132°C bei einer Dauer zwischen 30 und 60 Minuten. Somit können sowohl Streptokokken als auch Viren und Prionen zerstört werden und ein Übertragen von Krankheiten von einem Patienten zum anderen ausbleiben. Dadurch, dass die Sendeeinrichtung als solches autoklavierbar ist, kann die Medizinvorrichtung als Ganzes selbst ebenfalls autoklaviert werden.

In einer weiteren Ausführungsform ist die Medizinvorrichtung abgesehen von der Sendeeinrichtung frei von elektrischen stromführenden Bauteilen.

Somit müssen von der elektronischen Vorrichtung keine elektrischen und somit stromleitenden Kabel zu einer Energieversorgung oder einer Abfrageelektronik geführt werden. Auch kann auf eine gesonderte Sendeeinheit, welche einen Schaltzustand mittels einer aktiven Stromversorgung sendet, verzichtet werden, sodass das Gewicht der medizinischen Vorrichtung reduziert werden kann. Dies erleichtert den Umgang durch einen Bediener. Insbesondere wenn bei komplizierten Operationen über einen längeren Zeitpunkt ein Sauger oder dergleichen an einer Stelle verbleiben muss, ist dies für den Operateur oder das entsprechende Hilfspersonal vorteilhaft.

Um insbesondere auch im Fall des Nicht-Bedienens eine definierte Schalterstellung einzunehmen, können das erste Schaltelement und das zweite Schaltelement über einen Federelement derart gekoppelt sein, dass beim Nichtbetätigen der Schaltelemente eine Schalterposition definiert geschaltet ist. Weiterhin können somit auch Push-Push-Mechaniken realisiert werden, bei denen bei einem einmaligen Drücken eine erste Schalterposition und beim zweiten Drücken die zweite Schalterposition definiert eingenommen wird.

Vorliegend können das erste Schaltelement und das zweite Schaltelement zueinander rotatorisch und/oder translatorisch beweglich ausgestaltet sein. So kann abhängig von den Bedürfnissen eines Bedieners die Schaltkonstruktion ausgestaltet werden. Somit kann der dadurch realisierte Schalter sowohl ein Schiebeschalter (translatorisch) als auch ein Drehschalter (rotatorisch) sein.

Für den Fall, dass beispielsweise die Medizinvorrichtung selbst die Schalteinrichtung bildet oder ein weiteres medizinisches Werkzeug oder Handhabungsgerät mit der Medizinvorrichtung verbunden werden soll, kann die Medizinvorrichtung eine Adaptereinrichtung zum Verbinden der Medizinvorrichtung mit dem weiteren medizinischen Werkzeug aufweisen. So kann beispielsweise ein Griffelement bereitgestellt werden, an welches beispielsweise ein elastischer Schlauch und/oder ein Rohr angeflanscht wird, welche nach einmaliger Betätigung entsorgt werden können. Das Griffstück selbst kann dann beispielsweise nach einem Autoklavieren für eine weitere Behandlung mit einem neuen Schlauch und/oder Rohr versehen werden und an einem anderen Patienten eingesetzt werden.

Um die Ansteckungsmöglichkeit mit Keimen noch weiter zu reduzieren, werden medizinische Instrumente wie beispielsweise Saugrohre und Versorgungsschläuche der Saugrohre als Wegwerfteile ausgestaltet. Um die Medizinvorrichtung beispielsweise mehrfach benutzen zu können, weist diese erfindungsgemäß eine Arretiereinrichtung auf, über die ein medizinisches Instrument mit der Medizinvorrichtung mechanisch verbindbar ist, sodass nach einem Verbinden eine form- und/oder kraftschlüssige Verbindung zwischen der Medizinvorrichtung und dem medizinischen Instrument vorliegt.

Die Arretiereinrichtung realisiert dabei beispielsweise im Verbindungsfall die Verbindung zwischen der Medizinvorrichtung, z.B. ein Griffstück mit Schalter, und dem medizinischen Instrument (z.B. Saugrohr) und ggf. zusätzlich mit dem Versorgungsschlauch, welcher an einer Pumpe angeordnet ist, welche mittels des Schalters an und ausgestellt wird. Die Medizinvorrichtung kann auch im Wesentlichen nur die Schalteinrichtung und die Arretiereinrichtung aufweisen und mit verschiedenen Instrumenten oder Instrumententeilen verbindbar sein. Auf diese Weise lässt sich die bevorzugt passive Schalteinrichtung an verschiedene Instrumente ansetzen, auch wenn diese selbst keine elektrischen Einrichtungen aufweisen. Die Instrumente lassen sich so flexibel und nach Bedarf mit einer Schalteinrichtung zum Schalten elektrischer Einrichtungen in dem medizinischen System versehen. In einer diesbezüglichen Ausgestaltung weist die Arretiereinrichtung eine Entriegelungseinheit auf, sodass die Arretiereinrichtung lösbar fest mit dem medizinischen Instrument verbindbar ist.

Um verschiedene Alternativen für eine Arretiereinrichtung bereitzustellen, kann die Arretiereinrichtung ein Verrastelement, ein Klemmelement, ein Clipelement und/oder eine Schiebeelement aufweisen, welche die form- und/oder kraftschlüssige Verbindung mit dem medizinischen Instrument realisieren. Dabei muss das medizinische Instrument nicht verändert werden und kann ohne großen Aufwand mit einer Schalteinrichtung versehen werden.

In einer weiteren Ausgestaltung weist oder weisen die Adaptereinrichtung und/oder die Arretiereinrichtung eine Positioniereinheit auf, die die medizinische Vorrichtung an einem verbundenen medizinischen Instrument ausrichtet. Somit kann eine eineindeutige Ausrichtung der Medizinvorrichtung und des verbundenen medizinischen Instruments gewährleitet werden. In einer einfachen Ausgestaltung wird dies durch eine Nase und zugeordnete Nasenaufhahme realisiert. Auch Asymmetrien in der Geometrie der Medizinvorrichtung und des medizinischen Instruments können die Positioniereinheit bilden.

Bevorzugt ist die Medizinvorrichtung ein Endoskop oder ein Sauger oder ein anderes minimal-invasives Instrument. Insbesondere bei einem Sauger kann dieser im Wesentlichen aus Kunststoff ausgebildet werden und keine weiteren elektrischen oder elektronischen Bauteile aufweisen.

In einem weiteren Aspekt der Erfindung wird die Aufgabe gelöst durch ein medizinisches System mit einer elektrischen Einrichtung und einer zuvor beschriebenen zugeordneten Medizinvorrichtung.

Somit kann einem Arzt oder einem medizinischen Fachpersonal ein System zur Verfügung gestellt werden, bei welchem von der Medizinvorrichtung aus einzelne Einheiten aus der Ferne an- oder ausgeschaltet werden können, wobei die Medizinvorrichtung selbst keine aktiven elektronischen Bauteile aufweist oder gar über Batterie- oder Stromversorgungsquellen und dergleichen verfügt.

In einem weiteren Aspekt wird die Verwendung eines RFID-Elements zum Schalten einer externen elektrischen Einrichtung durch Aktivieren oder Deaktivieren einer Abschirmeinrichtung offenbart.

Somit kann ein Schalter für ein elektronisches oder elektrisches Bauteil bereitgestellt werden, welcher selbst keine aktiven elektrischen Bauteile aufweist.

Auch ist dieses ein Konzept zum Beschalten von externen elektronischen oder elektrischen Geräten unter der Verwendung von RFID-Elementen.

Bei der vorliegenden Abschirmeinrichtung kann es sich um den oben beschriebenen faradayschen Käfig oder ein Teil dessen handeln. Wesentlich dabei ist, dass durch das Abschirmen oder Nicht-Abschirmen ein elektrisches Schalten realisierbar ist. Entsprechend werden insbesondere die zugeschalteten elektronischen oder elektrischen Einrichtungen mit einem das RFID-Element auslesenden Lesegerät verknüpft und die Schaltlogik entsprechend elektrisch oder elektronisch umgesetzt.

Im Weiteren wird die Erfindung anhand von Ausführungsbeispielen näher erläutert. Es zeigen
Figur 1a einen Teilausschnitt eines schematisch dargestellten Schalters mit abgeschirmten RFID-Element,
Figur 1b einen Teilausschnitt des Schalters in einem geöffnetem Zustand, wobei das RFID-Element abfragbar ist,
Figur 1c eine stark schematische und teiltransparente Darstellung des Schalters mit angeflanschtem Griffstück in einer Zwischenposition,
Figur 1d eine stark schematische und teiltransparente Darstellung des Schalters mit angeflanschtem Griffstück in einer geschlossenen Schalterstellung,

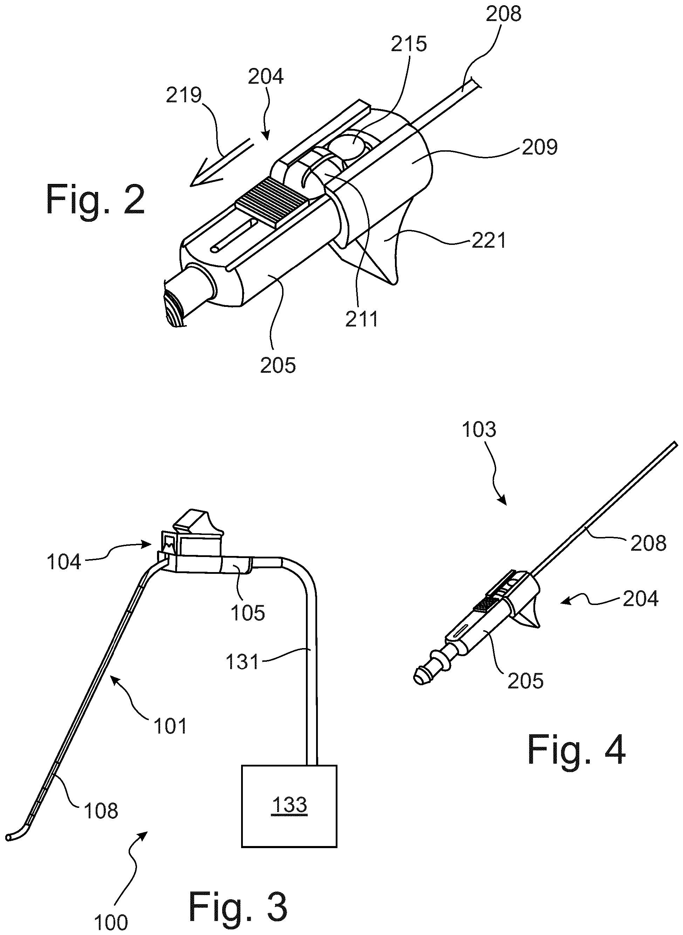

Figur 2 eine schematische dreidimensionale Darstellung eines Griffstücks mit angeflanschtem Schalter mit einem RFID-Element in einer Offenstellung,
Figur 3 eine stark schematische Darstellung eines medizinischen Systems mit einem Sauger mit einem starren Saugrohr und einem Griffstück mit angeflanschtem Schalter,
Figur 4 eine stark schematische Darstellung eines Saugers mit feststehendem Saugrohr und einem aufgeschobenen Schalter mit einem mit dem Schalter verbundenen Griffstück und
Figur 5 eine stark schematische Darstellung eines zweistückigen Schalters mit Griffstück und Schaltergehäuse.

Ein Schalter 104 weist ein Schaltergehäuse 109 und ein zu dem Schaltergehäuse 109 beweglich ausgestalteten Schiebeschlitten 111 auf. Zwischen dem Schiebeschlitten 111 und dem Schaltergehäuse 109 ist eine Spiralfeder 117 angeordnet, welche als Zugfeder ausgestaltet ist.

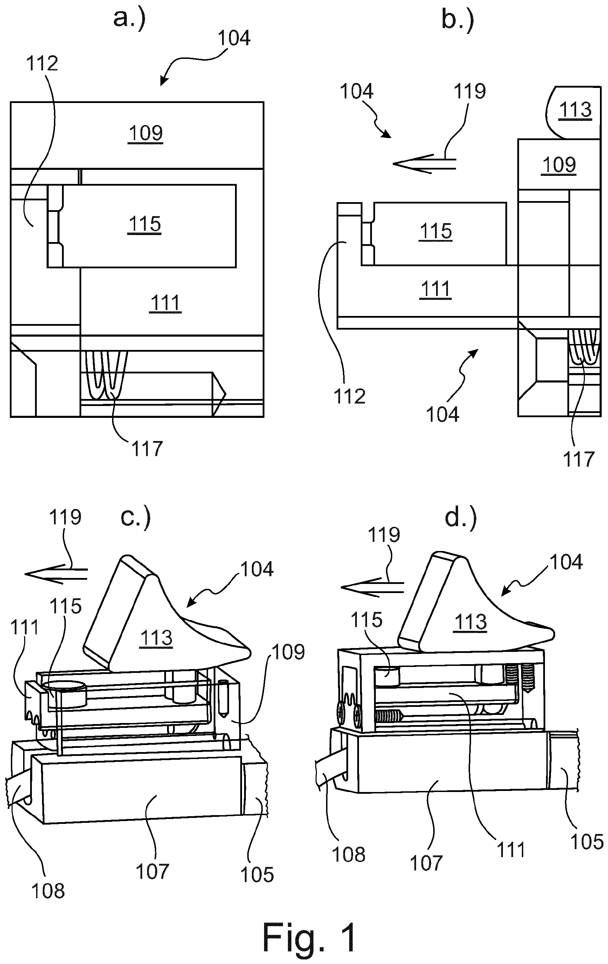

Auf dem Schiebeschlitten 111 ist ein passives RFID-Element fest angebracht. In einer Offenstellung (siehe Figur 1b) ist das RFID-Element 115 nach oben und zu den Seiten unbeschränkt zugänglich. Das Schaltergehäuse 109 weist ein engmaschiges Metallgitter (nicht dargestellt) auf, um welches herum Kunststoff gegossen ist, wobei der gegossene Kunststoff im ausgehärteten Zustand die Konturen des Schaltergehäuses 109 ausbildet. Im Bereich der vorderen Abdeckung 112 des Schiebeschalters 111 ist eine Metallplatte ebenfalls umgossen. Das feinmaschige Metallgitter in dem Schaltergehäuse 109 und die Metallplatte in der vorderen Abdeckung 112 des Schiebeschalters 111 bilden im geschlossenen Zustand (siehe Figur 1a) einen faradayschen Käfig.

Weiterhin ist an dem Schiebeschlitten 111 ein Schiebegriff 113 fest angeordnet. In dem Schiebegriff 113 ist ebenfalls eine Metallplatte eingearbeitet, sodass ein Schlitz (nicht dargestellt) in dem Schaltergehäuse 109, entlang dessen der Schiebegriff 113 beim Öffnen geführt wird, ebenfalls eine Abschirmwirkung ausbildet.

Der Schalter 104 ist auf einer Rohrführung 107 angeordnet. Die Rohrführung 107 bildet mit dem Griffstück 105 eine lösbare Rastverbindung aus. Sowohl durch das Griffstück 105, als auch durch die Rohrführung 107 ist das Rohr 108 geführt. Das Rohr 108 ist starr ausgeführt und bildet mit dem Griffstück 105 und dem Schalter 104 einen Sauger 101 mit starrem Saugrohr. Über das Griffstück 105 ist ein weiterer Versorgungsschlauch 131 mit einer Saugpumpe 133 verbunden.

Ein Lesegerät (nicht dargestellt) sendet permanent ein Abfragesignal. Im geschlossenen Fall (siehe Figur 1a) kann das RFID-Element 115 weder das Signal empfangen, noch ein Antwortsignal aussenden beziehungsweise das empfangene Signal modulieren. Aufgrund dessen, dass das Lesegerät keine Rückinformation erhält gibt das Lesegerät an die Saugpumpe das Stellsignal "Pumpe aus" weiter. Somit wird zusätzlich eine Geräuschquelle eliminiert.

Wird nun mittels des Schiebegriffs 113 der Schiebeschlitten 111 in Schieberichtung 119 verschoben, so wird das RFID-Element 115 freigelegt (siehe Figur 1b).

Das durch das Lesegerät ausgesandte Signal interagiert mit dem passiven RFID-Element 115 und das Lesegerät ermittelt sowohl die Identifikationsnummer, als auch die Tatsache dass das RFID-Element 115 abfragbar ist. Daraufhin wird das Stellsignal "Pumpe Ein" an die Saugpumpe gesandt. Die zugehörige Elektronik der Saugpumpe aktiviert aufgrund des Stellsignals den Motor, und über den Schlauch zum Griffstück 105 und den Schlauch 108 wird beispielsweise Körperflüssigkeit abgesaugt.

Sobald der Bediener den Schiebegriff 113 loslässt, zieht die Spiralfeder 117 den Schiebenschlitten 111 in das Schaltergehäuse 109 zurück. Aufgrund dessen, dass nun das RFID-Element 115 durch die Leseeinrichtung nicht mehr abfragbar ist, sendet die Leseeinrichtung an die Saugpumpe das Signal "Pumpe aus". Daraufhin deaktiviert die Elektronik der Saugpumpe die Pumpe und das Saugen wird unterbrochen.

In einer Alternative des Schalters 204 ist ebenfalls an einem Griffstück 205 ein Schaltergehäuse 209 angeordnet. Dieses Schaltergehäuse 209 weist einen vollständig aus Metall gefertigten Kern auf. In dem Schalter 209 ist das passive RFID-Element 215 fest angeordnet. In seitlichen Führungen (nicht dargestellt)des Schalters 209 ist der Schiebeknopf 211 angeordnet. Für einen besseren Halt ist an dem Schaltergehäuse 209 ein Fingergriff 221 angebracht.

In dem Schalterknopf 211 ist ein feinmaschiges Metallgitter eingebracht, welches in einem geschlossenen Fall mit dem in dem Schaltergehäuse 209 eingebrachten Metallkern eine vollständige Abschirmung mittels eines faradayschen Käfigs für das passive RFID-Element 215 bildet. Wird nun der Schiebeschalter 211 durch einen Bediener in Schieberichtung 219 bewegt, wird der obere Teil des passiven RFIDs 215 freigelegt. In diesem Fall ist das passive RFID 215 ebenfalls durch die Leseeinheit (nicht dargestellt) wie in der vorigen Alternative abfragbar, und eine Saugpumpe ansteuerbar.

Die Funktion des Schalters 204 wird beispielhaft an einem Sauger mit feststehendem Saugrohr 103 erläutert.

Das feststehende Saugrohr 208 ist durch den Schalter 204 und das Griffstück 205 zu einem Anschlussflansch geführt. Der Anschlussflansch wird über einen Schlauch mit der Saugpumpe verbunden. Wird nun durch einen Bediener der Schiebeknopf 211 in Schieberichtung 219 verschoben, wird das passive RFID-Element 215 freigelegt. Die Leseeinrichtung erhält ein Antwortsignal und überträgt an die Saugpumpe das Signal "Pumpe Ein", welches durch die Elektronik in der Saugpumpe ausgelesen wird und ein Aktivieren der Saugpumpe erfolgt. Über das feststehende Saugrohr 208 und die Zuführung zur Saugpumpe wird Flüssigkeit abgesaugt. Sobald ein Beenden des Saugvorganges erfolgen soll, schiebt der Bediener den Schiebeknopf 211 entgegen der Schieberichtung 219 bis zu einem Anschlag (nicht dargestellt). Aufgrund der Wirkung des faradayschen Käfigs der Metallgitter und Metallkerne, kann das passive RFID-Element 215 durch das Lesegerät nicht mehr abgefragt werden. Daraufhin erhält die Saugpumpe das Signal "Pumpe Aus", welche durch die Elektronik entsprechend umgesetzt wird.

Nach dem Einsatz der Sauger 101, 103 können diese vollständig mit dem Handgriffen 105 ,205 den Schaltern 104, 204 und dem feststehendem Rohr 208 in einem Autoklaven für die Sterilisation eingebracht werden. Nach der Sterilisation können die Schalter 204, 104 bei einem neuen Patienten eingesetzt werden.

In einer alternativen Ausgestaltung des Schalters (ergänzend erläutert anhand Figur 1a) ist der Schiebeschlitten eine feststehende Achse 111 und das Schaltergehäuse 109 ist rotierbar um die feststehende Achse 111 angeordnet. Das Schaltergehäuse 109 hat eine Öffnung (nicht dargestellt), welche durch Rotation in einer Stellung das passive RFID-Element freigibt. Somit ist ein Rotationsschalter realisiert.

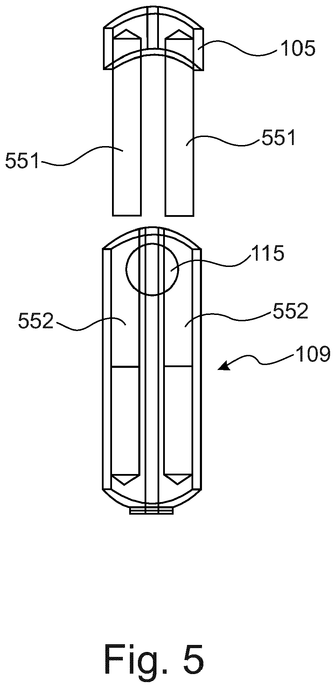

In einer weiteren alternativen Ausgestaltung des Saugers (siehe Figur 5) sind Griffstück 105 und Schaltergehäuse 109 mit RFID-Element 115 als getrennte Bauteile ausgeführt. Das Griffstück 105 ist fester Bestandteil des Saugrohrs (in Fig. 5 nicht dargestellt) und weist zwei Zapfen 551 auf. Korrespondierend dazu weist das Schaltergehäuse 109 zwei Zapfenaufnahmen 552 auf. Das Saugrohr und das Griffstück sind als Einmalinstrument ausgelegt. Das Schaltergehäuse 109 ist durch Autoklavieren mehrfach einsetzbar.

Wird nun auf das Griffstück 105 das Schaltergehäuse 109 aufgeschoben, bilden die Zapfen 551 und die Zapfenaufnahmen 552 eine Führung. Durch die korrespondierende Form ist ein falsches Aufstecken unmöglich. Im zusammengefügten Zustand wird eine Rastverbindung (nicht dargestellt) etabliert. An das Saugrohr wird nun der Schlauch der Saugpumpe angeschlossen. Der Arzt kann nun das Saugrohr verwenden. Nach dem Einsatz wird das Schaltergehäuse von Griffstück 105 und Saugrohr wieder abgezogen und das Griffstück und das Saugrohr entsorgt. Das Schaltergehäuse 109 wird autoklaviert und steht danach wieder zur Verfügung.

Für alle Beispiele gilt, dass elektrische Geräte wie beispielsweise Saugpumpen mittels vorliegenden Schaltern gesteuert werden können, obwohl Saugrohr, Schlauch, Griffstück, Rohrführung, Schaltergehäuse und Schiebeschlitten weder elektrische Leitungen noch elektrische Energiequellen aufweisen. Insbesondere können medizinische Instrumente, die nicht elektrisch angeschlossen oder mit elektrischen Leitungen versehen sind, wie z.B. Endoskope, Sauger, Skalpelle oder Fasszangen, mit einem Schalter ausgerüstet werden, ohne in den Instrumenten extra elektrische Leitungen und Anschlüsse vorsehen zu müssen.

### Bezugszeichenliste

- 100: Medizinisches System
- 101: Sauger mit flexiblem Schlauch
- 103: Sauger mit feststehendem Saugrohr
- 104: Schalter
- 105: Griffstück
- 107: Rohrführung
- 108: Saugrohr
- 109: Schaltergehäuse
- 111: Schiebeschlitten / feststehende Achse
- 112: Vordere Abdeckung des Schiebeschlittens
- 113: Schiebegriff
- 115: Passives RFID
- 117: Spiralfeder
- 119: Schieberichtung
- 131: Versorgungsschlauch
- 133: Saugpumpe
- 204: Schalter
- 205: Griffstück
- 215: Passives RFID
- 208: feststehendes Rohr
- 209: Schaltergehäuse
- 211: Schiebeknopf
- 219: Schieberichtung
- 221: Fingergriff551 Zapfen
- 552: Zapfenaufnahme

## Patentansprüche

1. Medizinvorrichtung (101, 103), welche insbesondere ein Bestandteil eines medizinischen Systems ist, mit einer Schalteinrichtung (104, 204) zum Aktivieren oder Deaktivieren einer von der Medizinvorrichtung räumlich beabstandeten elektrischen Einrichtung des medizinischen Systems, wobei die Schalteinrichtung ein erstes Schaltelement (109, 209) und ein zweites Schaltelement (111, 211) aufweist, welche zueinander beweglich ausgestaltet sind, sodass eine erste Schalterstellung und eine zweite Schalterstellung realisierbar sind, und das erste Schaltelement einen Teil eines faradayschen Käfigs oder einen faradayschen Käfig und das zweite Schaltelement eine Sendeeinrichtung (115, 215) aufweist, **dadurch gekennzeichnet, dass** das erste Schaltelement und das zweite Schaltelement derart zueinander ausgebildet sind, dass in der ersten Schalterstellung ein Aktivieren oder Deaktivieren der elektrischen Einrichtung des medizinischen Systems und in der zweiten Stellung ein zu der ersten Schalterstellung komplementäres Deaktivieren oder Aktivieren durch ein Abschirmen der Sendeeinrichtung mittels des Teils des faradayschen Käfigs oder mittels des faradayschen Käfigs erfolgt, und durch eine Arretiereinrichtung, über die ein medizinisches Instrument mit der Medizinvorrichtung mechanisch verbindbar ist, sodass nach einem Verbinden eine form- und/oder kraftschlüssige Verbindung zwischen der Medizinvorrichtung und dem medizinischen Instrument vorliegt.

2. Medizinvorrichtung nach Anspruch 1, **dadurch gekennzeichnet, dass** die Sendeeinrichtung frei von einer aktiven Energieversorgung ist.

3. Medizinvorrichtung nach einem der vorherigen Ansprüche, **dadurch gekennzeichnet, dass** die Sendeeinrichtung ein RFID-Element, insbesondere ein passives RFID-Element ist.

4. Medizinvorrichtung nach einem der vorherigen Ansprüche, **dadurch gekennzeichnet, dass** die Sendeeinrichtung eine Kodierung aufweist, welche durch die Sendeeinrichtung aussendbar ist, sodass die Medizinvorrichtung eindeutig identifizierbar ist.

5. Medizinvorrichtung nach einem der vorherigen Ansprüche, **dadurch gekennzeichnet, dass** die Sendeeinrichtung autoklavierbar ist, sodass nach einem Autoklavieren der Medizinvorrichtung die Sendeeinrichtung weiterhin funktionsgemäß einsetzbar ist.

6. Medizinvorrichtung nach einem der vorherigen Ansprüche, **dadurch gekennzeichnet, dass** das erste Schaltelement und das zweite Schaltelement über ein Federelement (117) derart gekoppelt sind, dass insbesondere bei einem Nichtbetätigen der Schaltelemente eine der Schalterpositionen definiert geschaltet ist.

7. Medizinvorrichtung nach einem der vorherigen Ansprüche, **dadurch gekennzeichnet, dass** das erste Schaltelement und das zweite Schaltelement zueinander rotatorisch und/oder translatorisch beweglich ausgestaltet sind.

8. Medizinvorrichtung nach einem der vorherigen Ansprüche, **dadurch gekennzeichnet, dass** die Arretiereinrichtung eine Entriegelungseinheit aufweist, sodass die Arretiereinrichtung lösbar fest mit dem medizinischen Instrument verbindbar ist.

9. Medizinvorrichtung nach einem der vorherigen Ansprüche, **dadurch gekennzeichnet, dass** die Arretiereinrichtung ein Verrastelement, ein Klemmelement, ein Clipelement und/oder eine Schiebeelement aufweist, welche die form- und/oder kraftschlüssige Verbindung beim Verbinden realisiert.

10. Medizinvorrichtung nach einem der vorherigen Ansprüche, **dadurch gekennzeichnet, dass** die Arretiereinrichtung eine Positioniereinheit aufweist, die die Medizinvorrichtung an dem medizinischen Instrument ausrichtet.

11. Medizinvorrichtung nach einem der vorherigen Ansprüche, **dadurch gekennzeichnet, dass** die Medizinvorrichtung ein Endoskop, ein Sauger oder ein anderes minimal-invasives Instrument ist.

12. Medizinisches System (100) mit einer elektrischen Einrichtung (133) und einer zugeordneten Medizinvorrichtung nach einem der Ansprüche 1 bis 11.

## Claims

1. Medical appliance (101, 103), which is in particular a constituent part of a medical system, with a switching device (104, 204) for activating or deactivating an electrical device of the medical system, said electrical device being situated spatially at a distance from the medical appliance, the switching device having a first switching element (109, 209) and a second switching element (111, 211), which are configured to be movable relative to each other such that a first switch position and a second switch position can be realized, and the first switching element having a part of a Faraday cage, or a Faraday cage, and the second switching element having a transmission device (115, 215), **characterized in that** the first switching element and the second switching element are configured relative to each other in such a way that, in the first switch position, the electrical device of the medical system is activated or deactivated, and, in the second position, a deactivation or activation complementary to the first switch position is effected by a shielding of the transmission device by means of the part of the Faraday cage, or by means of the Faraday cage, and by a locking device via which a medical instrument is mechanically connectable to the medical appliance such that, after they have been connected, there is a form-fit and/or force-fit connection between the medical appliance and the medical instrument.

2. Medical appliance according to Claim 1, **characterized in that** the transmission device is free of an active energy supply.

3. Medical appliance according to one of the preceding claims, **characterized in that** the transmission device is an RFID element, in particular a passive RFID element.

4. Medical appliance according to one of the preceding claims, **characterized in that** the transmission device has a coding which can be output by the transmission device, such that the medical appliance is uniquely identifiable.

5. Medical appliance according to one of the preceding claims, **characterized in that** the transmission device is autoclavable, such that, after the medical appliance has been autoclaved, the transmission device can still be used according to its function.

6. Medical appliance according to one of the preceding claims, **characterized in that** the first switching element and the second switching element are coupled via a spring element (117) in such a way that, in particular when the switching elements are not actuated, one of the switch positions is switched in a defined manner.

7. Medical appliance according to one of the preceding claims, **characterized in that** the first switching element and the second switching element are configured to be movable relative to each other in rotation and/or translation.

8. Medical appliance according to one of the preceding claims, **characterized in that** the locking device has an unlocking unit, such that the locking device is rigidly connectable to the medical instrument in a releasable manner.

9. Medical appliance according to one of the preceding claims, **characterized in that** the locking device has a latching element, a clamping element, a clip element and/or a slide element, which realizes the form-fit and/or force-fit connection during the connecting.

10. Medical appliance according to one of the preceding claims, **characterized in that** the locking device has a positioning unit, which aligns the medical appliance on the medical instrument.

11. Medical appliance according to one of the preceding claims, **characterized in that** the medical appliance is an endoscope, an aspirator or another minimally invasive instrument.

12. Medical system (100) having an electrical device (133) and an associated medical appliance according to one of Claims 1 to 11.

## Revendications

1. Dispositif médical (101, 103), qui est notamment un composant d'un système médical, ledit dispositif médical comprenant un moyen de commutation (104, 204) destiné à activer ou désactiver un moyen électrique du système médical qui est spatialement éloigné du dispositif médical, le moyen de commutation comportant un premier élément de commutation (109, 209) et un deuxième élément de commutation (111, 211) qui sont conçus pour être mobiles l'un par rapport à l'autre de façon à pouvoir occuper une première position de commutation et une deuxième position de commutation et le premier élément de commutation comportant une cage de Faraday ou une partie d'une cage de Faraday et le deuxième élément de commutation comportant un moyen d'émission (115, 215), **caractérisé en ce que**
le premier élément de commutation et le deuxième élément de commutation sont conçus de façon à effectuer, dans la première position de commutation, une activation ou désactivation du moyen électrique du système médical et, dans la deuxième position, une désactivation ou activation complémentaire de la première position de commutation par blindage du moyen d'émission à l'aide de la partie de la cage de Faraday ou à l'aide de la cage de Faraday, et par un moyen de blocage permettant de relier mécaniquement un instrument médical au dispositif médical de façon à établir, une fois que ceux-ci sont reliés, une liaison par complémentarité de formes et/ou en force entre le dispositif médical et l'instrument médical.

2. Dispositif médical selon la revendication 1, **caractérisé en ce que** le moyen émetteur est dépourvu d'une alimentation en énergie active.

3. Dispositif médical selon l'une des revendications précédentes, **caractérisé en ce que** le moyen émetteur est un élément RFID, en particulier un élément RFID passif.

4. Dispositif médical selon l'une des revendications précédentes, **caractérisé en ce que** le moyen émetteur comporte un code qui peut être émis par le moyen émetteur de façon à pouvoir identifier sans ambiguïté le dispositif médical.

5. Dispositif médical selon l'une des revendications précédentes, **caractérisé en ce que** le moyen émetteur est autoclavable de manière à pouvoir encore utiliser fonctionnellement le moyen émetteur après l'autoclavage du dispositif médical.

6. Dispositif médical selon l'une des revendications précédentes, **caractérisé en ce que** le premier élément de commutation et le deuxième élément de commutation sont accouplés par le biais d'un élément à ressort (117) de façon à commuter l'une des positions de commutation de manière définie, notamment lorsque les éléments de commutation ne sont pas actionnés.

7. Dispositif médical selon l'une des revendications précédentes, **caractérisé en ce que** le premier élément de commutation et le deuxième élément de commutation sont conçus pour être mobiles en rotation et/ou en translation l'un par rapport à l'autre.

8. Dispositif médical selon l'une des revendications précédentes, **caractérisé en ce que** le dispositif de blocage comporte une unité de déverrouillage de façon à pouvoir relier le dispositif de blocage solidairement et de manière amovible à l'instrument médical.

9. Dispositif médical selon l'une des revendications précédentes, **caractérisé en ce que** le dispositif de blocage comporte un élément d'encliquetage, un élément de serrage, un élément d'agrafage et/ou un élément coulissant qui réalise, lors de la liaison, la liaison par complémentarité de formes et/ou en force.

10. Dispositif médical selon l'une des revendications précédentes, **caractérisé en ce que** le moyen de blocage comporte une unité de positionnement qui oriente le dispositif médical sur l'instrument médical.

11. Dispositif médical selon l'une des revendications précédentes, **caractérisé en ce que** le dispositif médical est un endoscope, un moyen d'aspiration ou un autre instrument mini-invasif.

12. Système médical (100) comprenant un moyen électrique (133) et un dispositif médical associé selon l'une des revendications 1 à 11.
